Europäisches Patentamt

**European Patent Office**

Office européen des brevets

(11) Numéro de publication: **0 166 648**
**B1**

(12) **FASCICULE DE BREVET EUROPEEN**

(45) Date de publication du fascicule du brevet:
**24.08.88**

(21) Numéro de dépôt: **85401132.7**

(22) Date de dépôt: **10.06.85**

(51) Int. Cl.⁴: **C 10 L 1/02**

(54) **Procédé de valorisation d'essences oléfiniques par éthérification.**

(30) Priorité: **18.06.84 FR 8409623**

(43) Date de publication de la demande:
**02.01.86 Bulletin 86/1**

(45) Mention de la délivrance du brevet:
**24.08.88 Bulletin 88/34**

(84) Etats contractants désignés:
**BE DE FR GB NL**

(56) Documents cités:
**US - A - 3 902 870**

(73) Titulaire: **INSTITUT FRANCAIS DU PETROLE, 4, Avenue de Bois-Préau, F-92502 Rueil-Malmaison (FR)**
Titulaire: **ELF FRANCE, 137, Rue de l'Université, F-75007 Paris (FR)**

(72) Inventeur: **Torck, Bernard, 33, rue Vauthier, F-92100 Boulogne-sur-Seine (FR)**
Inventeur: **Amigues, Pierre, rue Felin, F-69340 Francheville (FR)**
Inventeur: **Weill, Jérôme, 16, route des Tourelles, F-69005 Lyon (FR)**
Inventeur: **Gueguen, Claude, rue de Chapoly, F-69540 Irigny (FR)**
Inventeur: **Llinares, Michel, 52, rue des Collonges Bat. D., F-69230 Saint-Genis-Laval (FR)**
Inventeur: **Bourgognon, Henri, 3, chemin de la Pomme, F-69160 Tassin-la-Demi-Lune (FR)**

(74) Mandataire: **Colas des Francs, Jean, Institut Français du Pétrole 4, Avenue de Bois Préau, F-92502 Rueil-Malmaison (FR)**

## Description

Dans la constitution d'un mélange d'essence (gasoline pool) les essences oléfiniques provenant des unités de craquage, de viscoréduction ou des coking, constituent une part importante des essences automobiles. Toutefois, d'une manière générale ces essences oléfiniques ont des indices d'octane relativement faibles, et il a été suggéré d'en augmenter leur qualité octane par éthérification de certaines des oléfines présentes par le méthanol. En effet, les fractions légères de ces essences oléfiniques contiennent des oléfines tertiaires telles que les isoamylènes, les isohexènes et les isoheptènes qui peuvent réagir facilement avec des alcools tels que le méthanol pour donner des éthers méthyliques.

Cette éthérification se fait en général avec des catalyseurs acides et plus particulièrement avec des résines échangeuses d'ions telles que les résines sulfoniques.

Le traitement des ces essences par le méthanol en présence de résine sulfonique permet d'augmenter leurs indices d'octane; par ailleurs, il diminue leur teneur en oléfines principalement en oléfines tertiaires et permet de valoriser du méthanol en supercarburant, sans aucun des problèmes dus au méthanol lorsqu'il est introduit directement dans les essences. En effet, le méthanol libre augmente la tension de vapeur de l'essence et conduit à des séparations de phase lorsque de l'eau est introduite dans les réservoir et les circuits de distribution.

C'est pour cette dernière raison que les législations des principaux pays n'autorisent l'introduction de méthanol libre qu'en présence de co-solvants qui suppriment les problèmes de demixtion.

La réaction entre le méthanol et les oléfines tertiaires est équilibrée et il est en général difficile d'obtenir des taux de conversion proches de 100%. Les taux de conversion des oléfines tertiaires sont d'autant moins élevés que le poids moléculaire de l'oléfine est plus grand.

Ainsi, s'il est possible dans le cas de l'isobutène d'atteindre par simple passage sur le catalyseur des conversions de l'ordre de 93 à 98%, dans le cas des isoamylènes, comme il est décrit dans le brevet français 2 411 881, les taux de conversion dans des conditions comparables à celles employées pour l'obtention de l'éther méthyl butylique tertiaire (MTBE), sont d'au plus 50 à 60 %- environ. Ce brevet montre qu'en recyclant au réacteur une partie des fractions légères, contenant les isoamylènes n'ayant pas réagi, obtenue par distillation de l'effluent du réacteur, il est alors possible d'atteindre un taux de conversion égal au maximum à environ 70%, si l'on désire rester dans des conditions opératoires économiquement raisonnables du fait du coût élevé de la distillation.

Un tel schéma de procédé ne permet pas d'éliminer l'excès de méthanol restant dans l'effluent du réacteur et qui se retrouve donc en majeure partie à l'état libre dans l'essence.

De manière à éliminer complètement le méthanol en excès, contenu dans le mélange obtenu par éthérification d'un mélange contenant des oléfines tertiaires en $C_4$ et $C_5$, il a été suggéré dans le brevet US 4 204 077, d'enlever le méthanol se trouvant dans l'effluent du réacteur par une extraction avec un solvant tel que l'éthylène glycol.

D'après les auteurs de ce brevet, ce solvant est préféré à l'eau qui, lors de l'extraction du méthanol, entraîne une partie des éthers présents dans l'effluent du réacteur.

Le document US-A-3 902 870 décrit un procédé d'amélioration de la qualité d'une fraction d'hydrocarbures $C_5^+$ bouillant dans la gamme des essences, contenant des composés oléfiniques par éthérification par un excès de méthanol. Il est mentionné que la coupe traitée est une coupe essence et non pas une coupe plus légère telle qu'une coupe $C_4^-$. Ce document ne précise nulle part la composition des coupes $C_5^+$ utilisées et on ne trouve aucune indication du fait que le problème rencontré lors du lavage à l'eau des produits obtenus par éthérification d'un mélange d'hydrocarbures contenant des oléfines à 4 et à 5 atomes de carbone ne se présentera plus si le mélange d'hydrocarbures ne contient pas d'hydrocarbures à 4 atomes de carbone.

L'objet de la présente invention, est de produire une coupe d'hydrocarbure $C_5^+$ à indice d'octane élevé.

Ce procédé de valorisation de coupes oléfiniques renfermant des hydrocarbures à 5 atomes de carbone et plus par molécule et notamment des isoamylènes, et ne renfermant substantiellement pas d'hydrocarbure à 4 atomes de carbone, c'est-à-dire moins de 5% en poids environ et de préférence moins de 1 % en poids par rapport au poids total de la coupe, comprend les étapes suivantes:

a) l'essence oléfinique est envoyée dans une zone d'éthérification où est réalisée la réaction entre ladite coupe et du méthanol en vue d'obtenir un effluent renfermant de l'éther méthyl amylique tertiaire,

b) ledit effluent obtenu à l'étape (a) est envoyé dans une zone d'extraction, dans laquelle on extrait à l'eau au moins la majeure partie du méthanol n'ayant pas réagi et on recueille une fraction débarrassée d'au moins la majeure partie du méthanol et contenant la majeure partie de l'éther méthyl amylique tertiaire.

c) l'extrait aqueux obtenu à l'étape (b) est fractionné en vue d'obtenir (α) une fraction enrichie en méthanol et appauvrie en eau et (β) une fraction appauvrie en méthanol et enrichie en eau.

d) ladite fraction enrichie en méthanol est recyclée au moins en partie à la zone d'éthérification et ladite fraction enrichie en eau est recyclée au moins en partie à la zone d'extraction.

Ledit procédé permet de valoriser les coupes oléfiniques d'hydrocarbures telles que les essences légères oléfiniques à condition qu'elles ne contiennent substantiellement pas d'hydrocarbure en $C_4$, par exemple une coupe $C_5$–$C_7$, et en particulier une essence légère de craquage catalytique contenant essentiellement des hydrocarbu-

res en $C_5$, $C_6$ et $C_7$ et renfermant entre autre des isoamylènes tels que le 2-méthyl-1-butène et le 2-méthyl-2-butène, des isohexènes tels que le 2,3-diméthyl-1-butène, le 2,3-diméthyl-2-butène, le 2-méthyl-2-pentène, le 2-méthyl-1-pentène, le 3-méthyl-2-pentène et le 2-éthyl-1-butène, et des heptènes tertiaires.

Pour atteindre des conversions suffisantes, on emploie une quantité de méthanol telle que le rapport molaire méthanol sur la somme des oléfines éthérifiables c'est-à-dire essentiellement oléfines tertiaires, contenues dans la coupe soit supérieur ou égal à 1:1 et de préférence compris entre 1:1 et 20:1, ce rapport étant de manière la plus préférée compris entre 2:1 et 6:1, à l'entrée de la zone d'éthérification.

La quantité d'eau utilisée dans la zone d'extraction représente avantageusement, en poids, 0,4 à 3 parties (de préférence 0,7 à 1,2 parties) pour 1 partie de la charge d'essence oléfinique. Dans ces conditions, la désactivation du catalyseur, au cours du temps, demeure faible. Avec des quantités d'eau inférieures aux proportions précitées, on observe une désactivation notable.

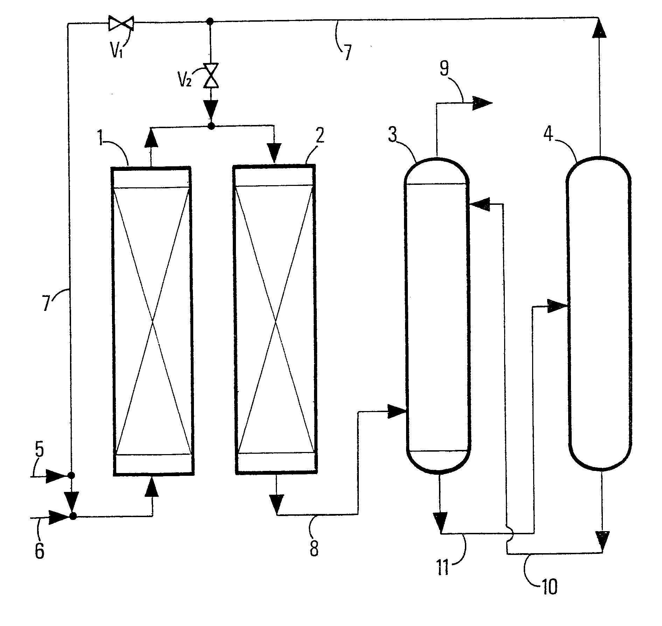

Le schéma de procédé, illustré par la figure unique, montre en plus qu'il est possible d'éthérifier par le méthanol des essences oléfiniques et d'enlever le méthanol en excès par simple lavage à l'eau et sans avoir recours à une distillation de l'effluent de réacteur, qui est une opération coûteuse.

Ce schéma illustre une des dispositions possibles propre à l'exécution du procédé.

Dans un tel procédé, l'essence oléfinique est introduite par la ligne (6) dans la zone d'éthérification qui d'une manière préférée est constituée par une batterie de 2 réacteurs (1 et 2) mis en série et contenant un catalyseur acide tel que les résines échangeuses d'ions, par exemple du type sulfonique, telle que celle décrite dans US – 3 037 052.

Le méthanol est introduit par la ligne (5) et mélangé à l'essence avant d'entrer dans le réacteur (1). L'effluent des réacteurs est introduit par la ligne (8) dans une colonne d'extraction à l'eau (3) marchant à contre-courant d'hydrocarbure et d'eau. L'essence éthérifiée débarrassée du méthanol, sort par la ligne (9) pour être envoyée au pool carburant. L'eau entre à la colonne d'extraction par la ligne (10). Elle sort, chargée de méthanol en fond de colonne et est envoyée par la ligne (11) à la colonne de distillation (4) pour séparer le méthanol qui sort en tête et est recyclé par la ligne (7) à travers la vanne $V_1$ à l'entrée de la zone d'éthérification.

Dans une forme de réalisation péférée, une partie au moins du méthanol est recyclé au réacteur (2) par la ligne (7) à travers la vanne $V_2$.

Ce schéma illustre un mode particulier d'exécution de l'invention. Diverses variantes peuvent être envisagées par l'Homme de l'Art, sans sortir du cadre de l'invention.

En particulier, la zone d'éthérification peut être formée par un seul réacteur ou par plus de deux réacteurs, le recyclage du méthanol non converti pouvant s'effectuer dans plusieurs d'entre eux.

De préférence, le recyclage du méthanol s'effectue au moins en partie, dans au moins un réacteur autre que le premier.

Les réacteurs de la zone d'éthérification sont associés de manière à ce que au moins deux d'entre eux soient en série, des associations de réacteurs dans lesquelles certains sont en parallèle pouvant être envisagées.

L'utilisation de ce procédé tel qu'illustré par la figure unique dans le cas de l'éthérification par le méthanol d'un mélange d'une essence oléfinique et d'une coupe $C_4$ de craquage n'est pas satisfaisant.

On constate en effet que le taux de conversion des oléfine tertiaires, isobutène, isoamylènes, isohexènes, diminue progressivement dans le temps et que l'eau servant au lavage de l'effluent des réacteurs se charge en alcool t-butylique ABT diminuant d'autant le rendement en éther [MTBE et éther méthyl amylique tertiaire (TAME)]. On attribue ces effets au fait que l'eau qui sert à extraire le méthanol entraîne par dissolution une partie du MTBE formé.

Lors de la distillation du méthanol dans la colonne (4), le MTBE sort en tête, entraînant de l'eau par azéotropie. Ce MTBE et l'eau qui sont alors introduits dans les réacteurs doivent contribuer à diminuer les performances du procédé.

On pourrait, dans le cas de l'éthérification d'un mélange d'essences oléfiniques et de coupe $C_4$, opérer comme décrit dans le brevet US 4 118 425, à savoir que l'eau ayant servi au lavage de l'effluent est éliminée, mais cela conduirait à une perte en MTBE et en méthanol.

Les auteurs de l'invention ont constaté par contre que dans le cas de l'éthérification par le méthanol d'une essence oléfinique ne contenant pas d'hydrocarbures en $C_4$, et donc par d'isobutène, ces inconvénients n'apparaissent pas lors d'un fontionnement en continu.

Ce schéma de procédé peut être appliqué aux essences insaturées, et en particulier aux essences de vapocraquage et aux essences légères de craquage catalytique.

Le point final de distillation des essences légères de craquage catalytique peut être compris entre environ 50 et environ 150 °C.

Après traitement, ces essences contiennent des éthers et sont essentiellement débarrassées de méthanol.

Les exemples suivants, illustrent l'invention sans en limiter la portée.

Exemple 1 (comparatif)

100 parts en poids d'une essence légère de craquage catalytique dont le point final de distillation est égal à 130 °C ne contenant pas d'hydrocarbures en $C_4$ et contenant essentiellement des hydrocarbures en $C_5$, $C_6$ et $C_7$ parmi lesquels 8% en poids d'isoamylènes, 6,5% d'isohexènes et 2,5% d'isoheptènes et 80 parts en poids d'une coupe $C_4$ contenant 25% en poids d'isobutène sont introduites par la ligne (6) dans le réacteur (1); 73,5 parts en poids de méthanol sont également introduites à l'entrée du réacteur de manière à ce que la rapport molaire méthanol sur la somme des isooléfines éthérifiables soit égal à 4. Ce méthanol est constitué d'une part par du méthanol frais, entrant par la ligne (5), nécessaire à la formation des éthers et d'autre part de l'excès de méthanol recyclé par la ligne (7) après distillation dans la colonne (4). La charge est introduite à 80 °C dans le réacteur (1) avec un débit tel que la vitesse volumique horaire (V.V.H.) soit égale à 6. L'effluent de ce réacteur est refroidi dans un échangeur de chaleur non représenté, pour être introduit à 70 °C dans le réacteur (2) avec un débit tel que la V.V.H. soit égale à 3. L'effluent du réacteur (2) est lavé par 250 parts d'eau dans la colonne d'extraction (3). L'eau contenant le méthanol, de 1 à 1,5% d'alcool t-butylique (ABT) et 0,6% de MTBE est distillée dans la colonne (4). En tête, sortent le méthanol et l'azéotrope MTBE–OH₂ (contenant 4% d'eau) qui sont recyclé au réacteur(s) par la ligne 7.

La composition de l'effluent sortant par la ligne (8) est donnée en fonction du temps dans le tableau 1.

Au bout de 300 heures de marche, la quantité d'éthers formée est plus faible et les taux de conversion de l'isobutène et des isoamylènes diminuent au cours du temps.

La conversion des oléfines tertiaires est donnée en pour cent.

Le produit sortant par la ligne (9), peut être utilisé directement pour la formation d'un mélange d'essence: sa composition est la même que celle de l'effluent sortant par la ligne (8) à l'exception près qu'il n'y a pratiquement plus de méthanol, ni d'ABT!

Exemple 2

100 parts en poids de l'essence légère de craquage catalytique de même point final et de même composition que dans l'exemple précédent sont introduites avec 27,8 parts de méthanol dans le réacteur (1).

Le rapport molaire sur la somme des isooléfines éthérifiables est égal à 4.

Le débit de charge, essence et méthanol, est tel que la V.V.H. soit égale à 6. La température à l'entrée du réacteur (1) est maintenu à 80 °C. L'effluent du réacteur est refroidi pour être introduit à 70 °C dans le réacteur (2) avec un débit tel que la V.V.H. soit égale à 3. L'effluent est lavé dans la colonne (3) avec 130 parts d'eau. L'eau contenant le méthanol est distillée dans la colonne (4). On sort en tête de colonne du méthanol essentiellement pur, qui est recyclé par la ligne (7) au réacteur (1). La composition de l'effluent sortant par la ligne (8) est donnée dans le tableau II. On constate que cette composition n'a pas changé après 300 heures de marche. On voit par ailleurs, que le taux de conversion des isoamylènes est plus élevé que dans l'exemple (1). La quantité d'éthers produits est également plus élevée. La composition du produit obtenu à la ligne (9) est la même que celle de l'effluent sortant par la ligne (8) à l'exception près qu'il n'y a pratiquement plus de méthanol.

Tableau 1

| Temps en heures Effluent poids | 30 | 100 | 300 |
|---|---|---|---|
| C₄ | 61,1 | 62,2 | 62,7 |
| C₅ | 27,4 | 28,0 | 28,2 |
| C₆ | 26,9 | 27,3 | 27,6 |
| C₇ | 35,5 | 35,6 | 35,7 |
| MTBE | 29,5 | 27,5 | 26,7 |
| TAME | 8,2 | 7,3 | 7,0 |
| *EtC₇ | 4,9 | 4,4 | 4,0 |
| *ETC₈ | 1,3 | 1,2 | 1,0 |
| CH₃OH | 58,5 | 59,7 | 60,2 |
| ABT | 0,15 | 0,25 | 0,3 |
| Conversion iC₄″% | 94,5% | 89% | 86,5% |
| Conversion iC₅″% | 70% | 63% | 60% |

*Les symboles EtC₇, EtC₈, signifient respectivement éther à 7 atomes et à 8 atomes de carbone.

Tableau II

| Temps en heures Effluent poids | 30 | 300 |
|---|---|---|
| C₅ | 26,6 | 26,70 |
| C₆ | 25,95 | 26,00 |
| C₇ | 35,25 | 35,85 |
| TAME | 9,32 | 9,20 |
| EtC₇ | 6,28 | 6,18 |
| EtC₈ | 1,66 | 1,58 |
| CH₃OH | 22,74 | 23,60 |
| Conversion iC₅″% | 80% | 79% |

**Revendications**

1. Procédé de valorisation d'une essence oléfinique renfermant des hydrocarbures à 5 atomes de carbone par molécule et notamment des isoamylènes et renfermant moins de 5% en poids d'hydrocarbures à 4 atomes de carbone, selon lequel:

a) l'essence oléfinique est envoyée dans une zone d'éthérification où est réalisée la réaction entre ladite coupe et du méthanol en vue d'obtenir un effluent renfermant de l'éther méthyl amylique tertiare,

b) ledit effluent obtenu à l'étape (a) est envoyé dans une zone d'extraction, dans laquelle on extrait à l'eau au moins la majeure partie du méthanol n'ayant pas réagit et on recueille une fraction débarrassée d'au moins la majeure partie du méthanol et contenant la majeure partie de l'éther méthyl amylique tertiaire,

c) l'extrait aqueux obtenu à l'etape (b) est fractionné en vue d'obtenir deux fractions: une fraction alpha enrichie en méthanol et appauvrie en eau et une fraction béta appauvrie en méthanol et enrichie en eau,

d) ladite fraction alpha enrichie en méthanol est recyclée au moins en partie à la zone d'éthérification et ladite fraction béta enrichie en eau est recyclée au moins en partie à la zone d'extraction!

2. Procédé selon la revendication 1 dans lequel l'essence oléfinique est une coupe en C$_5$–C$_7$.

3. Procédé selon la revendication 1 ou 2 dans lequel le méthanol contenu dans l'effluent d'éthérification est extrait par l'eau à contre-courant.

4. Procédé selon l'une des revendications 1 à 3 dans lequel la zone d'éthérification est constituée par au moins 2 réacteurs en série.

5. Procédé selon la revendication 4 dans lequel le méthanol recyclé est au moins en partie recyclé à l'entrée d'un autre réacteur que le premier.

6. Procédé selon l'une des revendications 1 à 5 dans lequel le rapport molaire méthanol-oléfines éthérifiables, à l'entrée de la zone d'éthérification est compris entre 1:1 et 20:1.

7. Procédé selon l'une des revendications 1 à 6 dans lequel le rapport molaire méthanol-oléfines éthérifiables, à l'entrée de la zone d'éthérification est compris entre 2:1 et 6:1.

8. Procédé selon l'une des revendications 1 à 7, dans lequel la quantité d'eau utilisée à l'étape (b) est de 0.4 à 3 parties en poids pour 1 partie en poids d'essence oléfinique.

9. Procédé selon l'une des revendications 1 à 7, dans lequel la quantité d'eau utilisée à l'étape (b) est de 0,7 à 1,2 parties en poids pour 1 partie en poids d'essence oléfinique.

## Patentansprüche

1. Verfahren zur Veredelung eines olefinischen Benzins, das Kohlenwasserstoffe mit 5 Kohlenstoffatomen je Molekül und insbesondere Iso- amylene und wenigstene 5 Gew.-% Kohlenwasserstoffe mit 4 Kohlenstoffatomen enthält, wobei

a) das olefinische Benzin in eine Veretherungszone geleitet wird, wo die Reaktion zwischen diesem Schnitt und Methanol derart durchgeführt wird, dass ein Abstrom erhalten wird, der tertiären Methylamylether enthält,

b) der aus Stufe (a) erhaltene Abstrom in eine Extraktionszone geleitet wird, in der mit Wasser wenigstens der Hauptteil des nicht umgesetzten Methanols extrahiert wird und eine Fraktion aufgefangen wird, die wenigstens zum grösseren Teil von Methanol befreit ist und hauptsächlich tertiären Methylamylether enthält,

c) der wässerige Extrakt der Stufe (b) derart fraktioniert wird, dass zwei Fraktionen erhalten werden, wobei eine Fraktion alpha mit Methanol angereichert und hinsichtlich Wasser verarmt ist und eine Fraktion beta an Methanol verarmt und mit Wasser angereichert ist,

d) die mit Methanol angereicherte Fraktion alpha wenigstens teilweise in die Veretherungszone recycliert wird und die mit Wasser angereicherte Fraktion beta wenigstene teilweise in die Extraktionszone recycliert wird.

2. Verfahren nach Anspruch 1, dadurch gekennzeichnet, dass das olefinische Benzin ein C$_5$– C$_7$-Schnitt ist.

3. Verfahren nach Anspruch 1 oder 2, dadurch gekennzeichnet, dass das im Abstrom der Veretherung enthaltene Methanol mit Wasser im Gegenstrom extrahiert wird.

4. Verfahren nach einem der Ansprüche 1 bis 3, dadurch gekennzeichnet, dass die Veretherungszone durch wenigstene zwei in Reihe geschaltete Reaktoren gebildet wird.

5. Verfahren nach Anspruch 4, dadurch gekennzeichnet, dass das Methanol wenigstens teilweise in den Einlass eines anderen Reaktors als dem ersten geleitet wird.

6. Verfahren nach einem der Ansprüche 1 bis 5, dadurch gekennzeichnet, dass das Molverhältnis Methanol/veretherbare Olefine am Einlass der Veretherungszone zwischen 1:1 und 20:1 liegt.

7. Verfahren nach einem der Ansprüche 1 bis 6, dadurch gekennzeichnet, dass das Molverhältnis Methanol/veretherbare Olefine am Einlass der Veretherungszone zwischen 2:1 und 6:1 liegt.

8. Verfahren nach einem der Ansprüche 1 bis 7, dadurch gekennzeichnet, dass die in Stufe (b) verwendete Menge Wasser bei 0,4–3 Gew,-Teilen je 1 Gew.-Teil olefinisches Benzin liegt.

9. Verfahren nach einem der Ansprüche 1 bis 7, dadurch gekennzeichnet, dass die in Stufe (b) verwendete Menge Wasser bei 0.7–1.2 Gew.-Teilen je 1 Gew.-Teil olefinisches Benzin liegt.

## Claims

1. A process for upgrading an olefinic gasoline containing hydrocarbons having 5 carbon atoms per molecule, particularly isoamylenes, and which contains less than 5% by weight of hydrocarbons with 4 carbon atoms, comprising:

a) feeding the olfinic gasoline to an etherification zone where said cut is reacted with methanol so as to obtain an effluent containing tert-amyl methyl ether,

b) feeding the effluent from step a) to an ex- traczion zone wherein at least the major part of the unreacted methanol is extracted with water and a fraction made free of at least the major part of tert-amyl methyl ether, is recovered,

c) fractionating the aqueous extract from step b), so as to obtain two fractions: α a fraction of increased methanol content and decreased water content, and (β) a fraction of decreased methanol content and increased water content,

d) recycling at least a portion of the fraction of increased methanol content to the etherification zone and recycling at least a portion of the fraction of increased water content to the extraction zone.

2. A process according to claim 1, wherein the olefinic gasoline is a C$_5$–C$_7$ cut.

3. A process according to claim 1 or 2, wherein the methanol contained in the etherification effluent is extracted with water in counter-current.

4. A process according to one of claims 1 to 3, wherein the etherification zone consists of at least 2 serially connected reactors.

5. A process according to claim 4, wherein the recycled methanol is at least partly recycled to the inlet of a reactor other than the first one.

6. A process according to one of claims 1 to 5, wherein the molar ratio methanol-etherifiable olefines, at the inlet of the etherification zone is from 1:1 to 20:1.

7. A process according to one of claims 1 to 6, wherein the molar ratio methanol-etherifiable olefines, at the inlet of the etherification zone is from 2:1 to 6:1.

8. A process according to one of claims 1 to 7, wherein the amount of water used in step (b) is from 0.4 to 3 parts by weight per part of olefinic gasoline charge.

9. A process according to one of claims 1 to 7, wherein the amount of water used in step (b) is from 0.7 to 1.2 parts by weight per part of olefinic gasoline charge.